# EUROPEAN PATENT APPLICATION

(11) **EP 0 744 392 A1**
(43) Date of publication of application: **27.11.1996**
(21) Application number: 96810309.3
(22) Date of filing: 15.05.1996
(51) Int. Cl.: C07C 45/51, C07C 205/45, C07C 225/22

(54) **A new one-pot process for the preparation of 7-aryl-2,6-disubstituted quinone methides**

(30) Priority: 26.05.1995 US 451382
(71) Applicant: CIBA-GEIGY AG, 4002 Basel (CH)
(72) Inventor: Evans, Samuel, Dr., 1723 Marly (CH); Nesvadba, Peter, Dr., 1723 Marly (CH); Allenbach, Stephan, 3186 Düdingen (CH)

(57) **Abstract**

The instant invention pertains to a new one-pot process for the facile preparation of 7-aryl-2,6-disubstituted quinone methides of formula I 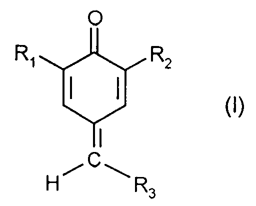
where R₁ and R₂ are independently alkyl of 1 to 18 carbon atoms, cycloalkyl of 5 to 12 carbon atoms or phenylalkyl of 7 to 15 carbon atoms, and R₃ is 2-, 3- or 4-pyridyl, 2- or 3-thienyl, 2- or 3-pyrryl, 2- or 3-furyl, aryl of 6 to 10 carbon atoms, or said aryl substituted by one to three alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms, alkoxycarbonyl of 2 to 8 carbon atoms, hydroxy, nitro, amino, cyano, carboxy, aminocarbonyl, chloro or mixtures of said substituents,
which are useful as polymerization inhibitors for vinyl aromatic and acrylic monomers.

## Description

The instant invention pertains to a new one-pot process for the preparation of 7-aryl-2,6-disubstituted quinone methides.
7-Aryl-2,6-disubstituted quinone methides are known in the prior art and a number of different methods for their preparation have been proposed.
United States Patent No. 4,032,547 describes an oxidation process for the preparation of quinone methides from hindered phenols using ferricyanide as the secondary oxidant in combination with persulfate as the primary oxidant.
German Offenlegungsschrift 2,734,239 discloses a process for the preparation of 2,6-di-tert-alkyl-4-alkylidene-2,5-cyclohexadienones by treatment of bis(3,5-di-tert-alkyl-4-hydroxybenzyl) sulfides with alkaline heavy metal compounds.
B. Koutek et al., Synthetic Communications, 6 (4), 305 (1976) describe a method for the preparation of 4-alkylidene-2,5-cydohexadien-1-ones by the facile cleavage of 4-hydroxybenzyl sulfonates with aqueous alkali. The sulfonates are prepared from the corresponding 4-hydroxybenzyl alcohols.
V. N. Komissarov et al., lzv. Akad. Nauk. Ser. Khim. 10, 2389 (1992) describes the preparation of photochromic and thermochromic Mannich bases from 3,5-di-tert-butyl-4-hydroxybenzaldehyde and 2-naphthols.
V. N. Komissarov et al., Zh. Organ. Khimii, 28 (3), 513 (1992) describes reactions of 2,6-dibutylphenol with aminals of aromatic o-hydroxy aldehydes which give Mannich bases in situ. These materials have photochromic properties in various organic solvents and thermochromic properties in alcohols. This chromism arises from the reversible dissociation of the Mannich base into the free amine and colored quinomethane derivatives.
H. Möhrle et al., Arch. Pharm. 316, 229 (1983) discloses that various p-substituted phenolic Mannich bases form alcohols and ethers when heated in aqueous alcohols in the presence of disodium ethylenediaminetetraacetic acid or acetic acid. The reaction proceed with amine elimination and subsequent addition of the solvent to the resulting quinone methide.
Filar et al., Tetrahedron Letters, 25, 9 (1960) teach the oxidation of phenols with silver oxide or lead dioxides to produce quinone methides.

The instant process is clearly superior to the processes disclosed in the prior art as outlined above. In the publications of V. N. Komissarov, the synthesis of Mannich bases are described, but the yields are generally poor (from 11 to 40%). In connection with thermochromic and photochromic properties exhibited by these bases the synthesis of some methylene quinone is described by treatment of the corresponding Mannich base with acetic anhydride.

The instant process relates to an unexpectedly high yield, one-pot process for the preparation of 2,6-disubstituted-4-benzylidene(and 4-substituted benzylidene)-2,5-cyclo-hexadienones by in situ formation of a Mannich base followed by subsequent elimination of an amine. This process has clear technical and economic advantages over the processes described in the Komissarov publications.

Additionally, the instant process is environmentally attractive since there is no involvement with heavy metals as required by the German Offen. and Filar methods. In the instant process, waste materials are reduced to a minimum and the amine by-products can be recycled.

The instant invention pertains to a one-pot process for the preparation of a 7-aryl-2,6-disubstituted quinone methide of formula I 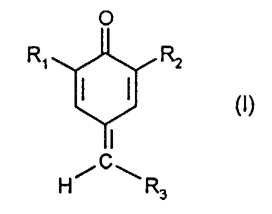 where
R₁ and R₂ are independently alkyl of 1 to 18 carbon atoms, cycloalkyl of 5 to 12 carbon atoms or phenylalkyl of 7 to 15 carbon atoms, and
R₃ is 2-, 3- or 4-pyridyl, 2- or 3-thienyl, 2- or 3-pyrryl, 2- or 3-furyl, aryl of 6 to 10 carbon atoms, or said aryl substituted by one to three alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms, alkoxycarbonyl of 2 to 8 carbon atoms, hydroxy, nitro, amino, cyano, carboxy, aminocarbonyl, chloro or mixtures of said substituents, which comprises
reacting in a first step a compound of formula II 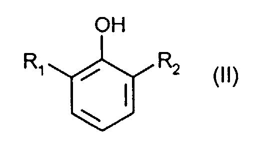 with a compound of formula III

   R₃-CHO (III)

   where R₁, R₂ and R₃ are defined above, and
a compound of formula IV

   R₄-NH-R₅ (IV)

   where R₄ and R₅ are independently hydrogen, alkyl of 1 to 12 carbon atoms, cyclohexyl, phenyl or benzyl, or R₄ and R₅ are together with the N atom to which they are attached are piperidino, morpholino or pyrrolidino,
to produce in situ a Mannich base of formula V 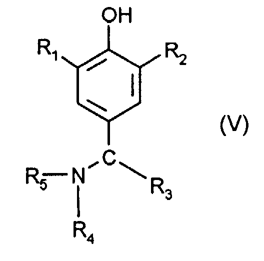 and
eliminating from the Mannich base in a second step, without first isolating said Mannich base, the compound of formula IV to give the compound of formula I.

Alkyl of 1 to 18 carbon atoms is linear or branched and is , for example, methyl, ethyl, propyl, isopropyl, butyl, isobutyl, sec.-butyl, tert.-butyl, pentyl, isopentyl, hexyl, heptyl, octyl, nonyl, decyl, dodecyl or octadecyl. Alkyl is, for example, C₁-C₁₂- , C₁-C₈-, C₄-C₁₂ , C₄-C₈- or C₁-C₄-alkyl.

Alkyl of 1 to 12 carbon atoms has the same meanings as given above for the corresponding number of carbon atoms.
Cycloalkyl of 5 to 12 carbon atoms is, for example, cyclopentyl, cyclohexyl, cycloheptyl, cyclooctyl or cyclodocecyl, especially, cyclopentyl and cyclohexyl.
Phenylalkyl of 7 to 15 carbon atoms is alkyl of 1 to 9 carbon atoms - as defined above - substituted with phenyl as, for example, benzyl, 2-phenylethyl, 3-phenylpropyl α-methylbenzyl or α,α-dimethylbenzyl, preferably benzyl.
Aryl of 6 to 10 carbon atoms is phenyl or α- or β-naphthyl, preferably phenyl. Substituted aryl is substituted by one to three, for example one or two, preferably one, substitutents.
Alkyl substituents of 1 to 8 carbon atoms are as defined above for the corresponding number of carbon atoms;
Alkoxy of 1 to 8 carbon atoms is linear or branched and is, for example methoxy, ethoxy, propoxy, isopropoxy, butoxy, isobutoxy, sec.-butoxy, tert.-butoxy, pentoxy, isopentoxy, hexyloxy, heptyloxy or octyloxy. Preferred is C₁-C₄-alkoxy.
Alkylthio of 1 to 8 carbon atoms is linear or branched and is, for example methylthio, ethylthio, propylthio, butylthio, tert.-butylthio, pentylthio, hexylhtio, heptylthio or octylthio. Preferred is C₁-C₄-alkylthio.
Alkylamino of 1 to 8 carbon atoms is linear or branched and means -NHR, wherein R is alkyl of 1 to 8 carbon atoms, as defined above for the corresponding number of carbon atoms. Examples are methylamino, ethylamino and butylamino.
Dialkylamino of 2 to 8 carbon atoms means -NRR', wherein R and R' independently of one another are alkyl of 1 to 7 carbon atoms (as defined above for the corresponding number of carbon atoms). Examples are dimethylamino, diethylamino, methylethylamino, dipropylamino or dibutylamino, preferably dimethylamino.
Alkoxycarbonyl of 2 to 8 carbon atoms means -(CO)O-C₁-C₇alkyl, wherein the C₁-C₇alkyl group is as defined above for the corresponding number of carbon atoms.
Examples for substituted aryl of 6 to 10 carbon atoms are hydroxyphenyl, nitrophenyl, aminophenyl, chlorophenyl, dichlorophenyl, tolyl, xylyl, ethylphenyl, methoxyphenyl, ethoxyphenyl, methylthiophenyl, dimethylaminophenyl, methyl-methoxyphenyl, especially, tolyl and methoxyphenyl.

Preferably, in the compound of formula I, R₁ and R₂ are the same; especially they are tert-butyl, tert-amyl, tert-octyl, cycloalkyl, α-methylbenzyl or α,α-dimethylbenzyl.

R₁ and R₂ for example are alkyl with 4 to 18, 4 to 12 or 4 to 8 carbon atoms.

Most preferably, R₁ and R₂ are tert-butyl, tert-amyl or tert-octyl.

Preferably, in the compound of formula I, R₃ is phenyl or phenyl substituted by nitro, cyano, dimethylamino, methoxy, alkyl of 1 to 4 carbon atoms, hydroxy or mixtures of said substituents.

Most preferably, R₃ is phenyl or phenyl substituted by a p-cyano, p-methoxy, p-dimethylamino, m-nitro or p-nitro group.

Most especially, R₃ is phenyl.

Preferably, R₄ and R₅ are the same and are alkyl of 1 to 8 carbon atoms or cyclohexyl, or R₄ and R₅ together with the N-atom to which they are attached are piperidino or morpholino.

Most preferably, R₄ and R₅ are the same and are alkyl of 1 to 4 carbon atoms, or together with the N-atom to which they are attached are piperidino or morpholino.

Most especially, R₄ and R₅ are each methyl or together with the N-atom to which they are attached are piperidino.

The instant process is run in one-pot, but entails two separate steps run sequentially without isolation of the Mannich base made in situ in the first step. The first step involves the preparation of the intermediate Mannich base followed by the second step which is an elimination reaction affording the instant compound of formula I in high yield and purity. The amine required to prepare the intermediate Mannich base is eliminated per se by a thermal elimination; or alternatively by an acid promoted elimination giving the acid salt of the original amine used to prepare the intermediate Mannich base; or alternatively by an anhydride promoted elimination giving a substituted amide of the anhydride used; or alternatively by quaternization with methyl iodide, methyl bromide or dimethyl sulfate and a subsequent Hofmann elimination reaction to give the compound of formula I and the original amine used to prepare the Mannich base.

The instant process is carried out at a temperature of 80°C-190°C, for example 130°C-160°C, preferably at 135°C-140°C.

The process can, for example, be carried out neat without any solvent, or using an inert high boiling solvent such as, for example, toluene, xylene, dichlorobenzene, paraffin oil, heptane, N,N-dimethyl-formamide, dioxane or N,N-dimethylacetamide. Preferably, the solvent used is toluene and/or xylene. In some cases, water can also be present.

When a thermal elimination is used for the second step, the temperature is preferably at 130°C-160°C and the elimination reaction is run at atmospheric pressure or under a slight vacuum. The free amine formed in this step can be recycled back to the first step of the instant process.

When an acid promoted elimination is used for the second step, the reaction is run, for example, at 135°C-140°C with a mineral acid such as hydrogen chloride, sulfuric acid or phosphoric acid; with an organic acid such as formic, acetic or propionic acid; with a Lewis acid such as aluminum trichloride or boron trifluoride; with supported acid catalysts such as acidic earths such as Fulcate® 22B or Fulmont®; or with silica gel. The amine can be regenerated from the amine salt and recycled back to the first step of the instant process.

When an anhydride promoted elimination is used for the second step, the reaction is, for example, run at 80°C-85°C with an anhydride such as acetic anhydride or propionic anhydride. The substituted amide obtained such as N,N-dimethylacetamide can be reused as an industrial solvent.

The order of addition of components in the first step can be altered somewhat. Each component (the phenol, the aldehyde and the amine) can be mixed together to form the Mannich base in situ with the loss of water.

Alternatively, the aldehyde and the amine can be added together to form an aminal intermediate with the loss of water. To this can then be added the phenol to form the Mannich base in situ.

Or, finally, the phenol and aldehyde can be reacted together followed by the addition of amine, the loss of water and the formation of the Mannich base in situ.

The molar ratios of the components in the instant process are as follows: for each mole of phenol, the molar ratio of aldehyde is, for example, 0.8 to 1.2, and the molar ratio of the amine is, for example, 0.8 to 2.0.

Preferably, equimolar amounts of phenol and aldehyde are reacted with the molar ratio of amine being for each mole of phenol, 1.0 to 2.5; especially 1.0 to 2.0; or especially preferred 1.0 to 1.5 mole.

The following examples are meant for illustrative purposes only and are not to be construed as limiting the instant invention in any manner whatsoever.

### Example 1: 2,6-Di-tert-butyl-4-benzylidene-cyclohexa-2,5-dienone

To a solution of 23.7 g (0.28 mol) of piperidine, 106.1 g (1.0 mol) of benzaldehyde and 206.3 g (1.0 mol) of 2,6-di-tert-butylphenol in 20 ml of toluene is added slowly 70 g (0.82 mol) of piperidine over a one-hour period at 135°C-140°C. The reaction mixture is then heated for another three hours with a continuous separation of water occurring. The resulting Mannich base prepared in situ is diluted with 200 ml of xylene and hydrogen chloride gas is bubbled into the reaction mixture at about 140°C till a state of saturation is reached in about 45 minutes. The mixture is heated for another hour to ensure that the reaction is complete as seen by thin layer chromatography (tlc) and gas liquid chromatography (glc) tests. The piperidine hydrochloride formed is removed by filtration. The dark red filtrate obtained is washed thrice with 200 ml of water and finally stirred with 100 g of Kieselgur for 30 minutes. Removal of the Kieselgur by filtration and evaporation of the solvent afford 285.6 g of a dark red viscous oil which contains about 90% (glc) of the title compound. This product is purified further by distillation under vacuum (10⁻² bar) giving 253.4 g (86.1% yield) of a fraction boiling between 160°C-168°C which is 96% pure in glc. This yellow viscous product slowly crystallizes on standing at room temperature.

| Analysis: | | |
|---|---|---|
| Calcd for C₂₁H₂₆O: | C, 85.7; | H, 8.9. |
| Found: | C, 85.5; | H, 9.0. |

### Example 2: 2,6-Di-tert-butyl-4-benzylidene-cyclohexa-2,5-dienone

The reaction described in Example 1 is repeated up to the point where the Mannich base is prepared in situ.

At this point, 122.5 g (1.2 mol) of acetic anhydride is added to the reaction mixture with stirring at 110°C for 15 minutes. The crude product is then purified by distillation at 10⁻² bar to afford 267.3 g (90.8% yield) of the title compound having 97.5% purity by glc.

### Example 3: 2,6-Di-tert-butyl-4-benzylidene-cyclohexa-2,5-dienone

(A) In a 0.3 L reactor, 51.6 g (0.25 mol) of 2,6-di-tert-butylphenol, 26.5 g (0.25 mol) of benzaldehyde and 42.3 g (0.375 mol) of a 40% aqueous solution of dimethylamine are reacted at 140°C for six hours. The reaction mixture is treated directly with 38.3 g (0.375 mol) of acetic anhydride and heated for 15 minutes at 100°C-110°C. Toluene (200 ml) is added and the organic phase is washed twice with 100 ml portions of water. Evaporation of the solvent affords a product which contains 90.3% (by glc) of the title compound.
(B) Repeating the procedure of (A), but using 0.3 mol of aqueous dimethylamine affords a product which contains 87% (by glc) of the title compound.
(C) Using equimolar concentrations of reactants and carrying out the reaction for ten hours at 140°C with subsequent treatment of the Mannich base produced in situ with an equimolar quantity of acetic anhydride affords a product containing 85.7% (by glc) of the title compound.

### Example 4: 2,6-Di-tert-butyl-4-(4-methoxybenzylidene)-cyclohexa-2,5-dienone

An equimolar amount of p-methoxybenzaldehyde is substituted for the benzaldehyde and the reaction is carried out as described in Example 1 to the place where the Mannich base is formed in situ. The reaction mixture is then treated with acetic acid at 110°C for 90 minutes to give the title compound after workup in a yield of 76% as a yellow crystalline solid after recrystallization from methanol. The product is roughly 99% pure (by glc) and melts at 115°C-116°C.

### Example 5: 2,6-Di-tert-butyl-4-(4-dimethylaminobenzylidene)-cyclohexa-2,5-dienone

An equimolar amount of p-dimethylaminobenzaldehyde is substituted for the p-methoxybenzaldehyde and the reaction is carried out as described in Example 2. After workup, the title compound is obtained as a red powder melting at 175°C.

| Analysis: | | | |
|---|---|---|---|
| Calcd for C₂₃H₃₁NO: | C, 81.8; | H, 9.3; | N, 4.2. |
| Found: | C, 81.7; | H, 9.2; | N, 4.2. |

### Example 6: 2,6-Di-tert-butyl-4-(3-nitrobenzylidene)-cyclohexa-2,5-dienone

An equimolar amount of m-nitrobenzaldehyde is substituted for the p-methoxybenzaldehyde and the reaction is carried out as described in Example 2. After workup, the title compound is obtained as an orange powder melting at 118°C.

| Analysis: | | | |
|---|---|---|---|
| Calcd for C₂₁H₂₅NO₃: | C, 74.3; | H, 7.4; | N, 4.1. |
| Found: | C, 74.1; | H, 7.4; | N, 4.1. |

## Claims

1. A one-pot process for the preparation of a 7-aryl-2,6-disubstituted quinone methide of formula I 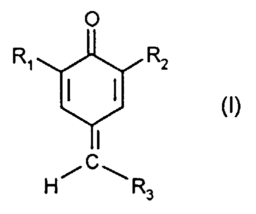 where
R₁ and R₂ are independently alkyl of 1 to 18 carbon atoms, cycloalkyl of 5 to 12 carbon atoms or phenylalkyl of 7 to 15 carbon atoms, and
R₃ is 2-, 3- or 4-pyridyl, 2- or 3-thienyl, 2- or 3-pyrryl, 2- or 3-furyl, aryl of 6 to 10 carbon atoms, or said aryl substituted by one to three alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms, alkoxycarbonyl of 2 to 8 carbon atoms, hydroxy nitro, amino, cyano, carboxy, aminocarbonyl, chloro or mixtures of said substituents, which comprises
reacting in a first step a compound of formula II 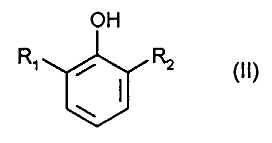 with a compound of formula III
R₃-CHO (III)
where R₁, R₂ and R₃ are defined above, and
a compound of formula IV
R₄-NH-R₅ (IV)
where R₄ and R₅ are independently hydrogen, alkyl of 1 to 12 carbon atoms, cyclohexyl, phenyl or benzyl, or R₄ and R₅ are together with the N atom to which they are attached are piperidino, morpholino or pyrrolidino,
to produce in situ a Mannich base of formula V 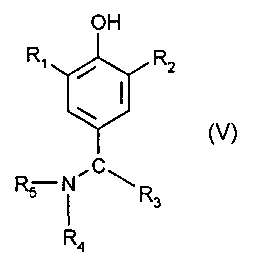 and eliminating from the Mannich base in a second step, without first isolating said Mannich base, the compound of formula IV to give the compound of formula I.

2. A process according to claim 1 where in the compound of formula I, R₁ and R₂ are the same.

3. A process according to claim 1 where in the compound of formula I, R₁ and R₂ are tert-butyl, tert-amyl, tert-octyl, cycloalkyl, α-methylbenzyl or α,α-dimethylbenzyl.

4. A process according to claim 1 where in the compound of formula I, R₃ is phenyl or phenyl substituted by nitro, cyano, dimethylamino, methoxy, alkyl of 1 to 4 carbon atoms, hydroxy or mixtures of said substituents.

5. A process according to claim 1 where in the compound of formula 1, R₃ is phenyl.

6. A process according to claim 1 where in the compound of formula IV, R₄ and R₅ are the same and are alkyl of 1 to 8 carbon atoms or cyclohexyl, or R₄ and R₅ together with the N-atom to which they are attached are piperidino or morpholino.

7. A process accroding to claim 1, wherein in the compounds of the formula I R₁ and R₂ are t-butyl and R₃ is phenyl or phenyl substituted with methoxy, dimethylamino or nitro.

8. A process according to claim 1 where in the compound of formula IV, R₄ and R₅ are the same and are alkyl of 1 to 4 carbon atoms, or together with the N-atom to which they are attached are piperidino or morpholino.

9. A process according to claim 1 wherein the reaction is run at a temperature of 80°C-190°C.

10. A process according to claim 11 wherein the reaction is run at a temperature of 135°C-140°C.

11. A process according to claim 1 wherein the process is carried out neat without any solvent, or using an inert high boiling solvent such as toluene, xylene, dichlorobenzene, paraffin oil, heptane, N,N-dimethylformamide, dioxane or N,N-dimethylacetamide.

12. A process according to claim 11 wherein the solvent used is toluene and/or xylene.

13. A process according to claim 1 wherein a thermal elimination is used for the second step, at a temperature of 130°C-160°C and the elimination reaction is run at atmospheric pressure or under a slight vacuum.

14. A process according to claim 1 wherein an acid promoted elimination is used for the second step, with the reaction being run at 135°C-140°C with a mineral acid, an organic acid, a Lewis acid or a supported acid catalyst.

15. A process according to claim 14 wherein the mineral acid is hydrogen chloride, sulfuric acid or phosphoric acid; the organic acid is formic, acetic or propionic acid; a Lewis acid is aluminum trichloride or boron trifluoride; a supported acid catalysts is an acidic earth, or a silica gel.

16. A process according to claim 1 wherein an anhydride promoted elimination is used for the second step, with the reaction being run at 80°C-85°C with an anhydride.

17. A process according to claim 16 wherein the anhydride is acetic anhydride or propionic anhydride.

18. A process according to claim 1 wherein for each mole of phenol of formula II, the molar ratio of aldehyde is 0.8 to 1.2, and the molar ratio of the amine is 0.8 to 2.0.
